# EUROPEAN PATENT SPECIFICATION

(11) **EP 0 534 568 B1**
(45) Date of publication and mention of the grant of the patent: **15.05.1996**
(21) Application number: 92202956.6
(22) Date of filing: 25.09.1992
(51) Int. Cl.: C12N 15/18, C07K 14/61, A61K 38/27, C12N 1/21

(54) **Human growth hormone analogues and their use**
Analoga von menschlichem Wachstumshormon und ihre Verwendung
Analogues de l'hormone de croissance humaine et leur utilisation

(30) Priority: 27.09.1991 IT MI912582
(43) Date of publication of application: 31.03.1993
(73) Proprietor: ENIRICERCHE S.p.A., I-20121 Milan (IT)
(72) Inventor: Grandi, Guido, I-20090 Segrate (Milan) (IT); Galli, Giuliano, I-20097 San Donato Milanese Milan (IT); Margarit Y Ros, Immaculada, I-20097 San Donato Milanese (Milan) (IT); Campagnoli, Susanna, I-20073 Codogno (Milan) (IT)
(74) Representative: Fusina, Gerolamo

(56) References cited:
- EP-A- 0 321 940
- CHEMICAL ABSTRACTS, vol. 85, no. 3, 19 July 1976, Columbus, Ohio, US; abstract no. 14263e, C H LI AND T A BEWLEY 'Human pituitary growth hormone: restoration of full biological activity by noncovalent interaction of two fragments of the hormone' page 78 ;
- CHEMICAL ABSTRACTS, vol.85, no.3, 19 July 1976, Columbus, Ohio, US; abstract no.14263e, C.H.LI and T.A.BEWLEY 'Human pituitary growth hormone: restoration of full biological activity by noncovalent interaction of two fragments of the hormone' page 78 & PROCEEDINGS OF THE NATIONAL ACADEMY OF SCIENCES OF USA, vol.73, no.5, 1976 Washington US, pages 1476-1479

## Description

This invention relates to human growth hormone mutants, means and methods for their preparation and their use in the therapeutic field.

The human growth hormone (hGH) is secreted within the pituitary gland during the entire life of an individual, and in a higher quantity during the preadult period. In its mature form it consists of a single polypeptide chain consisting of 191 amino acids, with a molecular weight of about 22,000 daltons.

The human growth hormone is characterised by multiple biological activity.

However its major characteristic is to promote the growth of the human body (skeleton, connective tissue, muscles, and viscera such as the liver, intestine and kidneys). The human growth hormone implements its action by interaction with specific receptors present in the cell membrane.

Up to a short time ago the only source of the hormone was the human pituitary gland extracted from cadavers, in that growth hormones of animal origin do not exhibit significant biological activity on man.

The poor availability of said hormone has therefore limited its application to the treatment of dysfunctions of the hypophysis, even though its effectiveness has been demonstrated in the treatment of pathological states such as burns, dystrophy, pseudoartrosis and bone fractures.

With the development of recombinant DNA techniques it has been possible to prepare the human growth hormone in more suitable quantities by fermentation processes using microorganisms transformed by a vector containing the coding gene for said hormone.

In particular, processes have been developed using proteolytic enzymes with cutting specificity for the removal of N or C terminal peptide extensions added to the hGH by genetic manipulation.

For example, patent application EP321940 describes a process in which the mature human growth hormone (191 amino acids) is expressed as the product (precursor) fused at its N-terminal amino acid residue (Phe) to the nonapeptide having the sequence Met-Glu-Glu-Leu-Met-Ile-Glu-Gly-Arg-, the purpose of which is to facilitate purification and improve the solubility of said product when expressed in B.subtilis cells.

The process comprises the removal of said nonapeptide by enzymatic cutting with the enzyme Factor Xa. Although the cutting specificity is high, mainly because the sequence -Ile-Glu-Gly-Arg-hGH is recognized and the peptide linkage between Arg and Phe (first hGH amino acid) is hydrolyzed, electrophoretic analysis of the hydrolysis product shows the appearance of two other protein species besides the mature hormone.

These species, which can represent 5-10% of the total proteins when the hydrolysis reaction is conducted to achieve 50% conversion of the precursor into hormone, are to be attributed to hydrolysis of the peptide linkage between Arg 134 and Thr 135 by Factor Xa.

The fact that the digestion of the precursor with Factor Xa is interrupted when about 50% hydrolysis is achieved and the need for further purification steps to completely separate the hGH from the enzymatic degradation products means that the final recovery yield of mature hGH is low.

It is also known that as in the case of polypeptides in general, when hGH is administered orally or intravenously it is degraded by the proteolytic enzymes present in the gastrointestinal tract and blood, which hydrolyze it rapidly into oligopeptides with no or only reduced biological activity.

For example, the hematic protease plasmin hydrolyzes the human growth hormone at Arg 134 with the production of two fragments 1-134 and 141-191 having reduced biological activity (0.3-14%) compared with that of natural (non-hydrolyzed) hGH [Li C.H. and Beweley (PNAS, 73: 1476-1479, 1976)].

In vivo demolition of the human growth hormone therefore reduces the quantity of hGH available and consequently requires the administration of large daily doses of this hormone.

It has now been found possible to overcome these drawbacks by preparing hGH mutants.

More specifically, the applicant has found, according to the teachings of the present invention, that by substituting the arginine amino acid residue in position 134 of the amino acid sequence of natural human growth hormone with a different amino acid residue, hGH mutants are obtained which are stable to enzymatic degradation without alteration to their biological activity.

These substitutions can be effected by mutagenizing the gene which codes for hGH by the site-specific mutagenesis method.

Notwithstanding the potentiality of this technique in engineering products with particular characteristics, various factors limit the general use of site-specific mutagenesis.

These limitations are due to insufficient knowledge of the relationship between structure and activity in polypeptides and their action mechanisms, so that the effect of modifying the amino acid sequence cannot be predicted and quantified in terms of physico-chemical and functional properties.

In this respect, the mutations introduced in the amino acid sequence of a polypeptide for altering one or more characteristics often induce an undesirable reduction in biological activity.

An object of the present invention is therefore to provide human growth hormone mutants with better stability towards enzymatic degradation than the natural hormone and with unaltered biological activity.

A further object of the present invention is to provide a DNA sequence containing the mutagenized structural gene of the human growth hormone which codes for a human growth hormone mutant having improved stability towards enzymatic degradation and with unaltered biological activity.

A further object of the present invention is to provide an expression cloning vector replicable in Bacillus subtilis comprising a DNA sequence containing the mutagenized structural gene of the human growth hormone which codes for a human growth hormone mutant having improved stability towards enzymatic degradation and with unaltered biological activity.

A further object of the present invention is to provide a Bacillus subtilis strain transformed by said vector.

A further object of the present invention is to provide a process for producing a human growth hormone mutant, comprising cultivating in a suitable culture environment a Bacillus subtilis strain transformed by an expression vector replicable in Bacillus subtilis comprising a DNA sequence containing the mutagenized human growth hormone structural gene which codes for a human growth hormone mutant having improved stability towards enzymatic degradation and with unaltered biological activity, lysing the cells and separating the soluble precursor of said mutant, hydrolyzing said soluble precursor with the enzyme Factor Xa and finally separating and purifying the obtained mature mutant from the culture medium.

A further object of the present invention is to use a human growth hormone mutant as the active principle in the preparation of pharmaceutical compositions.

A further object of the present invention is to provide pharmaceutical compositions which contain said mutants as active principle.

Further objects of the present invention will be apparent on reading the ensuing text and examples.

Specifically, human growth hormone mutants according to the present invention are characterised in that the amino acid residue arginine (Arg) in position 134 of the natural human growth hormone amino acid sequence is substituted with an amino acid residue chosen from the group consisting of L-alanine (Ala), L-leucine (Leu), L-threonine (Thr), L-phenylalanine (Phe), L-proline (Pro) and L-histidine (His).

Preferred according to the present invention are those human growth hormone mutants hGH in which the amino acid residue Arg 134 is substituted with the amino acid residue L-His or L-Leu.

The hGH mutants according to the present invention are prepared by a process comprising:
a) introducing a mutation into the structural gene coding for hGH using the site-specific mutagenesis technique;
b) cloning the mutagenized gene obtained in stage a) in an expression cloning vector replicable in Bacillus subtilis;
c) transforming a Bacillus subtilis with the recombinant vector obtained in stage b);
d) cultivating in a suitable culture environment the Bacillus subtilis strain transformed as in stage c);
e) lysing the Bacillus subtilis cells and separating the supernatant containing the soluble precursor of said mutant;
f) hydrolyzing the soluble precursor of said mutant with the enzyme Factor Xa; and finally
g) separating and purifying from the culture medium the obtained mature hGH mutant.

Of the various site-specific mutagenesis techniques which produce modifications targeted on a DNA sequence, the most widespread are those using single-helix synthetic oligonucleotides.

In one embodiment of the present invention the method described by Zoller M.J. and Smith M. (1982. Nucl. Acid. Res., 10, 6487-6500) was used, comprising:
1) inserting the hGH gene (target sequence) into a type M13 phage or into a plasmid derived therefrom and preparing it in the single strand form to be used as the template for the synthesis of the mutant gene;
2) synthesizing an oligonucleotide complementary to the sequence to be mutagenized with the exception of an internal portion which determines the mutation;
3) annealing the synthetic oligonucleotide to the template. The oligonucleotide acts as primer for the synthesis of the second modified strand;
4) by means of a polymerization step and in vitro ligation, reconstituting the double helix circular structure, of which one strand is the parental whereas the other carries the desired mutation;
5) using the double helix form to transform host cells made competent, to obtain a population of mutant and wild type clones;
6) selecting the mutant clones by hybridization with specific oligonucleotides, sequencing the clones obtained or restriction analysis.

Specifically, according to the process of the present invention, the DNA fragment EcoRI-HindIII of about 619 base pairs, comprising the nucleotide sequence (structural gene) coding for mature hGH fused at its 5'terminal to the sequence coding for the nonapeptide Met-Glu-Glu-Leu-Met-Ile-Glu-Gly-Arg, was isolated from the plasmid pSM274 (CBS 75.288) by digestion with the restriction enzymes EcoRI and HindIII.

This DNA fragment was then introduced into the phage M13mp9, previously digested with the aforesaid restriction enzymes, by ligation in a ligase mixture operating by known methods.

The ligase mixture was then used to transform Escherichia coli (E.coli) cells made competent as described by Dagert M. and Erlich (1979, Gene, 6: 23), the transformants being selected on suitable culture medium.

The single helix strand prepared from one of the positive plaques was used as the template for introducing the desired mutations. The following synthetic oligonucleotides were used for this purpose:

Ala
1 - GAA GAT GGA TCC CCC GCA ACT GGG CAG ATG
BamHI
2 - GAA GAT GGA TCC CCC NNN ACT GGG CAG ATG
BamHI

The first was synthesized for the purpose of substituting Arg134 with the amino acid Ala, and the second for obtaining random mutations which can virtually lead to substitutions with any other amino acid. In addition, both the oligonucleotides comprise a further mutation able to create BamHI in situ without altering the protein sequence in proximity to the desired substitution. This site allows rapid identification of the mutants by simple restriction analysis.

These oligonucleotides were synthesized by known methods using an automatic synthesizer.

The single helix was then annealed to the synthetic oligonucleotide to act as primer for the synthesis of the second modified strand.

After effecting the desired mutations, a polymerization step and in vitro ligation were used to reconstitute the circular double helix structure of the target sequence, of which one strand is the parental, while the other carries the desired mutation.

The DNA fragment of 619 bp containing the mutagenized hGH structural gene was then introduced into a cloning vector replicable in Bacillus subtilis, correctly positioning said fragment under the control of sequences which regulate its expression in the host strain.

Vectors suitable for the purpose can be chosen from plasmids available commercially or from authorized collection centres. The plasmid pSM214 ATCC 67320 was preferably used.

Correct insertion of the fragment of 619 bp containing the mutagenized hGH gene was verified by electrophoretic analysis on agarose gel (0.8%) after DNA digestion with the enzymes EcoRI and HindIII.

The plasmids containing the mutagenized genes Arg134→Ala. Arg134→Leu. Arg134→Thr. Arg134→Phe. Arg134→Pro and Arg134→His were named pSM386, pSM387, pSM388, pSM389, pSM390 and pSM391 respectively and were filed at the American Type Culture Center, where they received the following filing numbers: ATCC 68657, ATCC 68658, ATCC 68661, ATCC 68662. ATCC 68660, and ATCC 68659.

The resultant recombinant plasmids can be introduced into a host microorganism selected from the B.subtilis group, such as B.subtilis SMS 108 NRRLB-15898 or B.subtilis CBS 433.90, an asporigen strain.

In practice B.subtilis SMS 108 strains were transformed with said plasmids and cultivated in a suitable culture medium containing sources of carbon, nitrogen and mineral salts at a temperature of or about 37°C for the time necessary to obtain the desired product.

The cultures were lysed and the soluble proteins were recovered by centrifuging the cell lysates. Analysis of the supernatants obtained confirmed the presence of the hGH mutants.

The protein solutions were then purified by ion exchange chromatography and hydrophobic chromatography, collecting the fragments containing the soluble precursors of the hGH mutants, These solutions were concentrated and digested with the enzyme Factor Xa to remove the N-terminal nonapeptide from the precursor of said mutants.

The hydrolysis reaction was conducted at ambient temperature by adding purified Factor Xa (45 units; 2 mg/ml, 914 units/ml) to each solution.

Digesting the hGH mutants with Factor Xa allowed the mature hormone form (191 amino acids) to accumulate without giving rise to the production of the hydrolyzed form at the amino acid in position 134.

The mature mutants according to the present invention can be purified using one or more conventional chromatography methods.

The biological activity of the mutants according to the present invention was determined in vitro by a receptor assay using the method described by Tsushima T. and Friesen H.G., J. Clin. Endocrinol. Metab., 37: 337, (1973).

The results obtained showed that these mutants have a binding affinity to the growth hormone receptor comparable to that of unmodified hGH.

The hGH mutants according to the present invention have the following advantages over natural hGH:
- greater in vivo stability towards hematic protease action and consequently a greater duration of biological activity than natural hGH. This enables the daily dose of human growth hormone to be reduced;
- a higher yield from a mutant production process using Factor Xa for specific cutting of the nonapeptide bound to them.

The hGH mutants according to the present invention can be used as active principle in the formulation of pharmaceutical compositions for preparing dosage forms suitable for oral or parenteral administration.

The quantity of compound contained in a pharmaceutical composition can vary in accordance with different parameters such as the nature of the composition, including the particular compound contained in it, and the method of administration.

The mutants according to the present invention can be formulated in unit dosage forms depending on the method of administration.

In addition to the hGH mutants as active principle, the formulations of the present invention can contain stabilizers, adjuvants, preservatives, antioxidants, emulsifiers, antiflocculants, buffers and disaggregators chosen from those compatible with the compounds themselves and pharmacologically acceptable.

In the case of oral formulations, the pharmaceutical compositions can also contain flavouring as known in pharmaceutics.

### Brief description of the figures:

### Figure 1:

Nucleotide and amino acid sequences of hGH and mutants in the region subjected to site-specific mutagenesis.

### Figure 2:

Kinetics of hydrolysis of hGH and of the mutant Arg134-->Leu hGH by Factor Xa. Aliquots of the hydrolysis mixtures were fed at different times (0.2, 0.8 and 24 hours) onto acrylamide gel and after electrophoretic analysis were coloured with Comassie Blue.

Accumulation of the nicked form in the case of wild type hGH is apparent. This form is not present in the mutated protein.

The following experimental examples illustrate the invention but without limiting it thereto.

### EXAMPLE 1

### Cloning of the fragment EcoRI-HindIII of pSM274 containing the hGH gene

The plasmid pSM274 (CBS 75.288) (1 µg) and the replicative form of the phage M13mp9 (1 µg) were digested separately with the restriction enzymes EcoRI and HindIII (Boehringer) at 37°C for 1 hour. The reactions were conducted in 40 µl of a reaction mixture consisting of 10 mM Tris-HCL pH 8.0, 5 mM MgCl₂, 100 mM NaCl and 1 mM 2-mercaptoethanol in the presence of 4 units (U) of EcoRI and HindIII. After inactivating the enzymes at 65°C for 10 minutes, 2 µl of each reaction mixture were loaded onto agarose gel (0.8%) to verify complete digestion of the DNA.

12 µl of the pSM274 reaction mixture (300 ng of pSM274) and 4 µl of the M13mp9 reaction mixture (100 ng of M13mp9) were added to 40 µl of a ligase mixture containing 66 mM Tris-HCL pH 7.6, 10 mM MgCl₂, 10 mM dithiothreitol (DTT) and 1 mM ATP, and the DNA was ligated in the presence of 1 U of T4 DNA ligase (Boehringer) at 14°C for 14 hours.

An aliquot (10 ml) of the ligase mixture was used to transform E.coli 71/18 (BRL) cells rendered competent with calcium chloride as described by Dagert and Ehrlich (Gene, 6: 23, (1979)).

The transformants were selected on YT agar plates (8 g/l tryptone (DIPCO), 5 g/l yeast extract (DIFCO), 5 g/l NaCl) containing 40 µg/ml of X-GAL (5-bromo-4-chloro-3-indolyl-D-galactopyranoside) and 125 µg/ml of IPTG (isopropyl-β-D-thiogalactopyranoside).

By operating in the aforesaid manner numerous recombinant plaques (white) were obtained, easily distinguishable from the non-recombinant (blue).

One of these recombinant plaques was used to infect an E.coli 71/18 (BRL) culture from which the replicative DNA double helix form of the phage was subsequently extracted.

This DNA was then digested with the restriction enzymes EcoRI and HindIII under the aforesaid conditions to verify the presence of the EcoRI-HindIII fragment of 619 base pairs containing the hGH gene isolated from pSM274.

The single helix DNA form. prepared as described by Messing J. ("Methods in Enzymology" Vol. 101, 1983), was then used as template in site-specific mutagenesis experiments for substituting arginine in position 134.

### EXAMPLE 2

### Site-specific mutagenesis

Site-specific mutagenesis was effected by using the "Oligonucleotide-directed in vitro mutagenesis, 2nd version" system marketed by Amersham, under the conditions described by the supplier.

Two oligodeoxynucleotides with the following sequences were used as primers:

Ala
1 - GAA GAT GGA TCC CCC GCA ACT GGG CAG ATG
BamHI
2 - GAA GAT GGA TCC CCC NNN ACT GGG CAG ATG
BamHI

The first primer was synthesized for the purpose of substituting Arg134 with the amino acid Ala, and the second for obtaining random mutations which could virtually lead to substitutions with any other amino acid. In addition, both the primers comprised a further mutation able to create BamHI in situ without altering the protein sequence in proximity to the desired substitution. This site allows rapid identification of the mutants by simple restriction analysis.

The oligonucleotides were synthesized by known methods using the One Plus DNA Synthesizer (Beckman) and then phosphorylated at the 5' terminal in 30 µl of reaction mixture containing 100 mM Tris-HCL pH 8.0, 10 mM MgCl₂ 5 mM DTT, 0.1 mM ATP and 2 units of kinase polynucleotide (Boehringer). The reaction was conducted at 37°C for 45 minutes.

The reactions described in the mutagenesis kit, which can be summarized as follows, were then carried out:
1 - annealing between the phosphorylated primer and the single helix of the recombinant phage;
2 - in vitro synthesis of the double helix phagic DNA in the presence of polymerase, ligase and dTTP, dCTP, dGTP, dATP and the adenosylphosphorothioate analog;
3 - cutting the DNA helix, used as template, with the enzyme NciI followed by complete removal of this with hexonuclease III;
4 - synthesizing the double helix DNA in the presence of polymerase, ligase and dNTP;
5 - transforming competent cells of E.coli with the double helix DNA;
6 - initial identification of the mutants by restriction analysis of the replicative forms (double helix DNA) of the recombinant phages.

Five recombinant phages which were cut from the BamHI restriction enzyme within the hGH gene were further analyzed by sequencing the DNA using the method described by Sanger and Coulson (1979) (J. Mol. Biol., 94: 441). The primer used for sequencing had the sequence CTA GAG GAA GGC ATC CAA and hybridized on the hGH gene in proximity to the region coding for arginine in position 134.

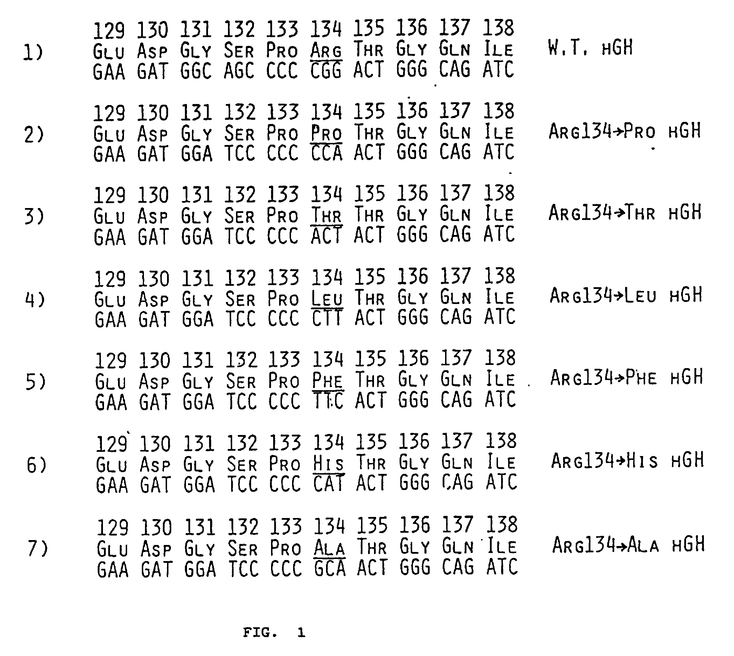

The sequences of the five DNAs are shown in Figure 1 and indicate clearly that the arginine was substituted with the amino acid residues Phe, Leu, Pro, Thr and His.

### EXAMPLE 3

### Transfer of mutations in the plasmid pSM274

The double helix DNAs (1.6 µg) of the mutagenized phages were separately digested with 2 units of EcoRI and HindIII under the aforesaid conditions. After verifying completion of cutting by electrophoretic analysis on agarose gel (0.8%), six ligase mixtures were prepared each containing 1.5 µg of each of the digested phagic DNAs and 0.5 µg of pSM274 previously digested with the same restriction enzymes. The ligation reaction was effected in 100 µl of reaction mixture having the initially described composition in the presence of 1 unit of T4 ligase, by incubation at 14°C for 14 hours.

The ligase mixtures were used to transform B.subtilis SMS108 cells (NRRLB-15898) made competent as described by Contente and Dubnau, (1979) (Mol. Gen. Genet. 167. 251). The transformants were selected on plates of VY agar culture medium (0.8% tryptone (DIFCO), 0.5% yeast extract (DIFCO), 0.5% NaCl) containing 5 µg/ml of kanamycin and 5 µg/ml of chloramphenicol.

The resultant clones resistant to both antibiotics were used for preparing plasmidic DNAs as described by Birnboim and Doly, Molecular Cloning (1979).

Verification of the correct insertion of the fragment of 619 bp carrying the mutated hGH gene was effected by electrophoretic analysis on agarose gel (0.8%) after DNA digestion with the enzymes EcoRI and HindIII.

The plasmids containing the mutagenized genes Arg134→Ala, Arg134→Leu, Arg134→Thr, Arg134→Phe, Arg134→Pro and Arg134→His were named pSM386, pSM387, pSM388, pSM389, pSM390 and pSM391 respectively.

### EXAMPLE 4

### Preparation and purification of hGH mutants

Single colonies of the six clones containing the plasmids carrying the Arg134 mutations were used to inoculate 250 ml of VY culture medium containing 5 µg/ml of chloramphenicol. After growing at 37°C overnight the cells were pooled for centrifuging at 5000 r.p.m. for 10 minutes, then resuspended in 50 ml of Tris-HCl 20 mM pH 7.5, again centrifuged and then resuspended in 10 ml of Tris-HCl 20 mM pH 7.5. 1 mM PMSF (phenylmethanesulphonylfluoride).

The cells were lysed by three successive passages in a French press at 18,000 psi and the soluble proteins were recovered by centrifuging the cell lysates for 30 minutes at 30,000 r.p.m. Analysis of aliquots (5 µl) of the obtained supernatants on SDS-polyacrylamide 12.5% gel confirmed the presence of hGH mutants.

The protein solutions were then fed into a Q-Sepharose^{R} Fast Flow (Pharmacia) column (1 x 7 cm) equilibrated in 20 mM Tris-HCl pH 6.5 at a flow rate of 15 ml/hour. The column was then washed with the equilibrium buffer and the proteins were eluted with 50 ml of a NaCl gradient from 0 to 0.15 M, collecting the fractions every 6 minutes (about 1.5 ml per fraction).

The fractions were analyzed by electrophoresis on SDS-polyacrylamide 12.5%.

The hGH mutants were eluted into 7 fractions. The pooled fractions (about 10 ml) had a total protein content of about 2.5-3.0 mg.

The solutions obtained after pooling the fractions were adjusted to a 1 M NaCl concentration and then fed into a Phenyl-Sepharose^{R} (Pharmacia) column (1 x 7 cm) equilibrated in 20 mM Tris-HCl pH 7.5, 1M NaCl at a flow rate of 15 ml/hour.

After washing the column with the equilibrium buffer the proteins were eluted with 20 mM Tris-HCl pH 7.5. The human growth hormone mutants were collected into 5 fractions. The pooled fractions (about 7.5 ml) had a total protein content of about 1.5-2.0 mg.

The solutions containing the six hGH mutants, adjusted to pH 8 with Tris 0.2 M, were concentrated in Centriprep 10 (AMICON) to a final volume of 0.5-0.6 ml before being digested with bovine Factor Xa enzyme for removing the N-terminal nonapeptide.

The hydrolysis reactions were conducted at ambient temperature by adding to each solution 50 µl of Factor Xa (2mg/ml) obtained by activating bovine Factor Xa, purified as described by N. Hashimoto et al. (J. Biochem. 97:1347, 1985) with viper venom immobilized on Sepharose^{R} 4B activated with BrCN.

The hydrolysis was followed by withdrawing 5 µl samples and examining them on SDS-polyacrylamide.

Under the experimental conditions adopted, the enzymatic reactions reached completion after about 24-30 hours.

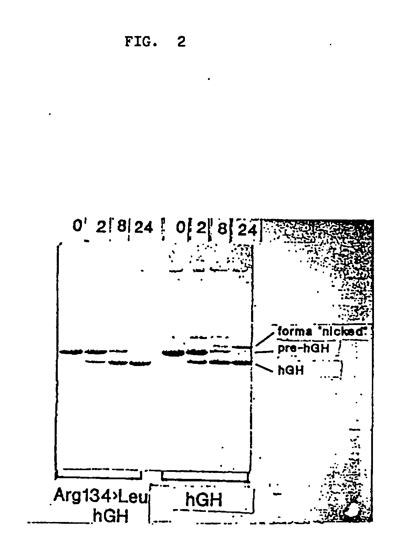

Digesting the hGH mutants with Factor Xa allowed accumulation of the mature form (191 amino acids) of the hormone, without giving rise to the production of the hydrolyzed form at the amino acid in position 134 (Figure 2).

The mutants were finally purified, after diluting the samples with 5 ml of 20 mM Tris-HCl pk 6.5 buffer, in a Q-Sepharose^{R} Fast Flow (Pharmacia) column (1 x 7 cm) equilibrated with the same dilution buffer.

The column was then washed with the equilibrium buffer and the proteins were eluted with 50 ml of a NaCl gradient from 0 to 0.15 M, collecting fractions every 6 minutes (about 1.5 ml per fraction).

The human growth hormone mutants were collected into 5 fractions (7.5 ml total volume), with a purity exceeding 95% (0.5-1.0 mg of total proteins).

### EXAMPLE 5

### Receptor assay

The object of this experiment was to evaluate the effect produced by individual amino acid substitutions in position 134 of the human growth hormone sequence on the capacity of the hormone to bind to its own receptor.

The assay was conducted on a fraction enriched in microsomal liver membranes isolated from a rabbit in a state of advanced pregnancy as described by Tsushima T. and Friesen H.G., "Radioreceptor Assay for Growth Hormone", J. Clin. Endocrinol. Metab.. 37: 337, )1973), using the ¹⁵I-hGH radioactive binder and mutant solutions for constructing standard curves.

The samples were incubated at ambient temperature for two hours in 50 mM Tris-HCl buffer. 15 mM CaCl₂ and 5 mg/ml of bovine serum albumin (BSA). pH 7.6. The binding reaction was blocked by adding to each test-tube 2.5 ml of sodium acetate pH 5.4 containing 0.1% of BSA.

The test-tubes contained about 70.000 counts per minute of ¹⁵I-hGH radioactive binder, 100 µl of hepatic microsomal suspension (250 µg of proteins) and both natural hGH and the mutant to be assayed at a final concentration of between 2.5 and 1000 ng/ml.

The results given in Table I below indicate that the Arg 134 substitutions of the hGH sequence do not substantially alter the tertiary hGH structure in that portion of the molecule which interacts with the membrane receptor.

**TABLE 1**

| Assayed product | IC50 (ng/ml)±S.E. | % of activity VS 212/5 |
|---|---|---|
| Ala134 | 25.51±1.36 | 74.6 |
| His134 | 20.01±1.07 | 95.2 |
| Leu134 | 20.47±0.88 | 87.0 |
| Phe134 | 22.50±4.59 | 79.1 |
| Thr134 | 21.24±1.56 | 83.8 |
| Pro134 | 23.45±1.45 | 81.2 |
| hGH | 19.05±1.41 | 100.0 |

IC50 is the quantity of displacer able to reduce the specific linkage with the receptor by 50%.

### SEQUENCE LISTING

SEQ ID NO. 1
   SEQUENCE TYPE: Peptide
   SEQUENCE LENGTH: 9 amino acids
   STRANDEDNESS: Single
   TOPOLOGY: Linear Met Glu Glu Leu Met Ile Glu Gly Arg 9
SEQ ID NO. 2
   SEQUENCE TYPE: Nucleotide
   SEQUENCE LENGTH: 30 base pairs
   STRANDEDNESS: Single
   TOPOLOGY: Linear
   PROPERTIES: Primer GAAGATGGAT CCCCCGCAAC TGGGCAGATG 30
SEQ ID NO. 3
   SEQUENCE TYPE: Nucleotide
   SEQUENCE LENGTH: 30 base pairs
   STRANDEDNESS: Single
   TOPOLOGY: Linear
   PROPERTIES: Primer GAAGATGGAT CCCCCNNNAC TGGGCAGATG 30
SEQ ID NO.4
   SEQUENCE TYPE: Nucleotide
   SEQUENCE LENGTH: 18 base pairs
   STRANDEDNESS: Single
   TOPOLOGY: Linear
   PROPERTIES: Primer CTAGAGGAAG GCATCCAA
SEQ ID NO. 5
   SEQUENCE TYPE: Nucleotide
   SEQUENCE LENGTH: 10 base pairs
   STRANDEDNESS: Single
   TOPOLOGY: Linear
   PROPERTIES: Primer

CTAGAGGAAG GCATCCAA

## Claims (Claims for the following Contracting State(s): AT, BE, FR, DE, GB, LU, NL, SE, CH, LI, DK, IE, MC, PT)

1. Human growth hormone mutants stable towards enzymatic degradation and with unaltered biological activity, characterised in that the amino acid residue arginine in position 134 of the natural human growth hormone amino acid sequence is substituted with at least one amino acid residue chosen from the group consisting of L-alanine. L-leucine. L-threonine. L-phenylalanine. L-proline and L-histidine.

2. A mutant as claimed in claim 1, wherein the amino acid residue arginine in position 134 is substituted with L-alanine.

3. A mutant as claimed in claim 1, wherein the amino acid residue arginine in position 134 is substituted with L-leucine.

4. A mutant as claimed in claim 1, wherein the amino acid residue arginine in position 134 is substituted with L-threonine.

5. A mutant as claimed in claim 1, wherein the amino acid residue arginine in position 134 is substituted with L-phenylalanine.

6. A mutant as claimed in claim 1, wherein the amino acid residue arginine in position 134 is substituted with L-proline.

7. A mutant as claimed in claim 1. wherein the amino acid residue arginine in position 134 is substituted with L-histidine.

8. A DNA sequence comprising the structural gene which codes for a human growth hormone mutant claimed in each of claims 1 to 7.

9. A cloning vector containing a DNA sequence comprising the structural gene which codes for a human growth hormone mutant claimed in each of claims 1 to 7.

10. The vector claimed in claim 9. chosen from the group consisting of expression plasmids replicable in Bacillus subtilis.

11. A Bacillus subtilis strain transformed with a plasmid according to claim 10.

12. Bacillus subtilis SMS108 (pSM 386) ATCC 68657.

13. Bacillus subtilis SMS108 (pSM 387) ATCC 68658.

14. Bacillus subtilis SMS108 (pSM 388) ATCC 68661.

15. Bacillus subtilis SMS108 (pSM 389) ATCC 68662.

16. Bacillus subtilis SMS108 (pSM 390) ATCC 68660.

17. Bacillus subtilis SMS108 (pSM 391) ATCC 68659.

18. A human growth hormone mutant as claimed in claim 1. obtained by a process comprising:
a) cultivating in a suitable culture environment a Bacillus subtilis strain transformed by a plasmid in accordance with claim 11:
b) lysing the cells and separating the soluble precursor of said mutant in which the N-terminal phenylalanine amino acid residue is fused to the nonapeptide having the sequence Met-Glu-Glu-Leu-Met-Ile-Glu-Gly-Arg-:
c) hydrolyzing said soluble precursor with the enzyme Factor Xa: and finally
d) separating and purifying the mature human growth hormone mutant obtained in this manner.

19. A human growth hormone mutant in accordance with claim 18. wherein Bacillus is Bacillus subtilis SMS108 (pSM 386) ATCC 68657.

20. A human growth hormone mutant in accordance with claim 18. wherein Bacillus is Bacillus subtilis SMS108 (pSM 387) ATCC 68658.

21. A human growth hormone mutant in accordance with claim 18, wherein Bacillus is Bacillus subtilis SMS108 (pSM 388) ATCC 68661.

22. A human growth hormone mutant in accordance with claim 18, wherein Bacillus is Bacillus subtilis SMS108 (pSM 389) ATCC 68662.

23. A human growth hormone mutant in accordance with claim 18, wherein Bacillus is Bacillus subtilis SMS108 (pSM 390) ATCC 68660.

24. A human growth hormone mutant in accordance with claim 18, wherein Bacillus is Bacillus subtilis SMS108 (pSM 391) ATCC 68659.

25. A human growth hormone mutant in accordance with claim 1, for use as active principle in the preparation of pharmaceutical compositions.

26. A pharmaceutical composition suitable for treating dysfunctions of the hypophysis, burns, dystrophy, pseudoartrosis and bone fractures, containing as active principle a human growth hormone mutant in accordance with claim 1.

## Claims (Claims for the following Contracting State(s): ES, GR)

1. Process for the preparation of human growth hormone mutants stable towards enzymatic degradation and with unaltered biological activity, in which the amino acid residue arginine in position 134 of the natural hormone amino acid sequence is substituted with at least one amino acid residue chosen from the group consisting of L-alanine, L-leucine, L-threonine, L-phenylalanine, L-proline and L-histidine, said process comprising:
a) introducing a mutation into a structural gene coding for human growth hormone by site specific mutagenesis technique;
b) cloning the mutagenized gene obtained in stage a) in an expression cloning vector replicable in Bacillus subtilis;
c) transforming a Bacillus subtilis with the recombinant vector obtained in stage b);
d) cultivating in a suitable culture medium a Bacillus subtilis strain transformed as in stage c);
e) lysing the Bacillus subtilis cells and separating the supernatant containing the soluble precursor of said mutant;
f) hydrolysing the soluble precursor of said mutant with the enzyme Factor Xa; and finally
g) separating and purifying from the culture medium the mature human growth hormone obtained in this manner.

2. A process for the preparation of a mutant as claimed in claim 1, wherein the amino acid residue arginine in position 134 is substituted with L-alanine.

3. A process for the preparation of a mutant as claimed in claim 1, wherein the amino acid residue arginine in position 134 is substituted with L-leucine.

4. A process for the preparation of a mutant as claimed in claim 1, wherein the amino acid residue arginine in position 134 is substituted with L-threonine.

5. A process for the preparation of a mutant as claimed in claim 1, wherein the amino acid residue arginine in position 134 is substituted with L-phenylalanine.

6. A process for the preparation of a mutant as claimed in claim 1, wherein the amino acid residue arginine in position 134 is substituted with L-proline.

7. A process for the preparation of a mutant as claimed in claim 1, wherein the amino acid residue arginine in position 134 is substituted with L-histidine.

8. Process for the preparation of human growth hormone mutant according to claim 1, wherein the mutagenesis in stage a) comprises:
1) inserting the hGH gene (target sequence) into a type M13 phage or into a plasmid derived therefrom and preparing it in the single strand form to be used as the template for the synthesis of the mutant gene;
2) synthesizing an oligonucleotide complementary to the sequence to be mutagenized with the exception of an internal portion which determines the mutation;
3) annealing the synthetic oligonucleotide to the template; the oligonucleotide acting as primer for the synthesis of the second modified strand;
4) by means of a polymerisation step and in vitro litigation, reconstituting the double helix circular structure, of which one strand is the parental whereas the other carries the desired mutation;
5) using the double helix form to transform host cells made competent, to obtain a population of mutant and wild type clones;
6) selecting the mutant clones by hybridization with specific oligonucleotides, sequencing the clones obtained or restriction analysis.

9. The process for the preparation of human growth hormone mutant according to claim 1, wherein the cloning vector in stage b) is selected from the group consisting of expression plasmids replicable with Bacillus subtilis.

10. The process for the preparation of human growth hormone mutant according to claim 1, wherein the Bacillus subtilis in stage d) is Bacillus subtilis SMS 108 (pSM 386) ATCC 68657.

11. The process for the preparation of human growth hormone mutant according to claim 1, wherein the Bacillus subtilis in stage d) is Bacillus subtilis SMS 108 (pSM 387) ATCC 68658.

12. The process for the preparation of human growth hormone mutant according to claim 1, wherein the Bacillus subtilis in stage d) is Bacillus subtilis SMS 108 (pSM 388) ATCC 68661.

13. The process for the preparation of human growth hormone mutant according to claim 1, wherein the Bacillus subtilis in stage d) is Bacillus subtilis SMS 108 (pSM 389) ATCC 68662.

14. The process for the preparation of human growth hormone mutant according to claim 1, wherein the Bacillus subtilis in stage d) is Bacillus subtilis SMS 108 (pSM 390) ATCC 68660.

15. The process for the preparation of human growth hormone mutant according to claim 1, wherein the Bacillus subtilis in stage d) is Bacillus subtilis SMS 108 (pSM 391) ATCC 68659.

16. A method for the preparation of a pharmaceutical composition suitable for treating dysfunctions of the hypophysis, bums, dystrophy, pseudoartrosis and bone fractures, containing as active principle a human growth hormone mutant obtained according to claim 1.

## Patentansprüche (Patentansprüche für folgende(n) Vertragsstaat(en): AT, BE, FR, DE, GB, LU, NL, SE, CH, LI, DK, IE, MC, PT)

1. Gegenüber einem Enzymabbau beständige Mutanten von menschlichem Wachstumshormon mit unveränderter biologischer Aktivität, dadurch gekennzeichnet, daß der Aminosäurerest Arginin in Position 134 der Aminosäuresequenz des natürlichen menschlichen Wachstumshormons durch wenigstens einen Aminosäurerest, ausgewählt aus der aus L-Alanin, L-Leucin, L-Threonin, L-Phenylalanin, L-Prolin und L-Histidin bestehenden Gruppe, ersetzt ist.

2. Mutante nach Anspruch 1, worin der Aminosäurerest Arginin in Position 134 durch L-Alanin ersetzt ist.

3. Mutante nach Anspruch 1, worin der Aminosäurerest Arginin in Position 134 durch L-Leucin ersetzt ist.

4. Mutante nach Anspruch 1, worin der Aminosäurerest Arginin in Position 134 durch L-Threonin ersetzt ist.

5. Mutante nach Anspruch 1, worin der Aminosäurerest Arginin in Position 134 durch L-Phenylalanin ersetzt ist.

6. Mutante nach Anspruch 1, worin der Aminosäurerest Arginin in Position 134 durch L-Prolin ersetzt ist.

7. Mutante nach Anspruch 1, worin der Aminosäurerest Arginin in Position 134 durch L-Histidin ersetzt ist.

8. Eine DNA-Sequenz, umfassend das Strukturgen, das für eine Mutante des menschlichen Wachstumshormons, wie in einem der Ansprüche 1 bis 7 beansprucht, codiert.

9. Klonierungsvektor mit einem Gehalt an einer DNA-Sequenz, die das Strukturgen umfaßt, welches für eine Mutante des menschlischen Wachstumshormons, wie in einem der Ansprüche 1 bis 7 beansprucht, codiert.

10. Vektor nach Anspruch 9, ausgewählt aus der Gruppe, die aus in Bacillus subtilis replizierbaren Expressionsplasmiden besteht.

11. Mit einem Plasmid nach Anspruch 10 transformierter Bacillus subtilis-Stamm.

12. Bacillus subtilis SMS108 (pSM 386) ATCC 68657

13. Bacillus subtilis SMS108 (pSM 387) ATCC 68658

14. Bacillus subtilis SMS108 (pSM 388) ATCC 68661

15. Bacillus subtilis SMS108 (pSM 389) ATCC 68662

16. Bacillus subtilis SMS108 (pSM 390) ATCC 68660

17. Bacillus subtilis SMS108 (pSM 391) ATCC 68659

18. Mutante des menschlichen Wachstumshormons, wie in Anspruch 1 beansprucht, erhältlich nach einem Verfahren, das umfaßt:
a) Kultivieren eines Bacillus-subtilis-Stammes, der durch ein Plasmid gemäß Anspruch 11 transformiert ist, in einem geeigneten Kulturmedium;
b) Auflösen der Zellen und Abtrennen des löslichen Precursors der Mutante, worin der N-endständige Phenylalaninaminosäurerest an das Nonapeptid mit der Sequenz Met-Glu-Glu-Leu-Met-Ile-Glu-Gly-Arg- fusioniert ist;
c) Hydrolysieren dieses löslichen Precursors mit dem Enzym Faktor Xa;
und schließlich
d) Abtrennen und Reinigen der solcherart erhaltenen reifen Mutante von menschlichem Wachstumshormon.

19. Mutante von menschlichem Wachstumshormon nach Anspruch 18, worin der Bacillus Bacillus subtilis SMS108 (pSM 386) ATCC 68657 ist.

20. Mutante von menschlichem Wachstumshormon nach Anspruch 18, worin der Bacillus Bacillus subtilis SMS108 (pSM 387) ATCC 68658 ist.

21. Mutante von menschlichem Wachstumshormon nach Anspruch 18, worin der Bacillus Bacillus subtilis SMS108 (pSM 388) ATCC 68661 ist.

22. Mutante von menschlichem Wachstumshormon nach Anspruch 18, worin der Bacillus Bacillus subtilis SMS108 (pSM 389) ATCC 68662 ist.

23. Mutante von menschlichem Wachstumshormon nach Anspruch 18, worin der Bacillus Bacillus subtilis SMS108 (pSM 390) ATCC 68660 ist.

24. Mutante von menschlichem Wachstumshormon nach Anspruch 18, worin der Bacillus Bacillus subtilis SMS108 (pSM 391) ATCC 68659 ist.

25. Mutante von menschlichem Wachstumshormon nach Anspruch 1, zur Verwendung als Wirkstoff in der Herstellung von pharmazeutischen Zusammensetzungen.

26. Pharmazeutische Zusammensetzung, die zur Behandlung von Dysfunktionen der Hypophyse, Verbrennungen, Dystrophie, Pseudoarthrose und Knochenbrüchen geeignet ist, mit einem Gehalt an einer Mutante von menschlichem Wachstumshormon gemäß Anspruch 1 als aktivem Wirkstoff.

## Patentansprüche (Patentansprüche für folgende(n) Vertragsstaat(en): ES, GR)

1. Verfahren zur Herstellung von gegenüber einem Enzymabbau beständigen Mutanten von menschlichem Wachstumshormon mit unveränderter biologischer Aktivität, worin der Aminosäurerest Arginin in Position 134 der Aminosäuresequenz des natürlichen Hormons durch wenigstens einen Aminosäurerest, ausgewählt aus der aus L-Alanin, L-Leucin, L-Threonin, L-Phenylalanin, L-Prolin und L-Histidin bestehenden Gruppe, ersetzt ist, welches Verfahren umfaßt:
a) Einführen einer Mutation in ein für das menschliche Wachstumshormon codierendes Strukturgen durch ortspezifische Mutagenese -Technik;
b) Klonieren des in Stufe a) erhaltenen mutagenisierten Gens in einem in Bacillus subtilis replizierbaren Klonierungsvektor;
c) Transformieren eines Bacillus subtilis mit dem in Stufe b) erhaltenen rekombinanten Vektor;
d) Kultivieren eines wie in Stufe c) transformierten Bacillus subtilis-Stammes in einem geeigneten Kulturmedium;
e) Auflösen der Bacillus subtilis-Zellen und Abtrennen des Überstandes, der den löslichen Precursor der Mutante enthält;
f) Hydrolysieren des löslichen Precursors dieser Mutante mit dem Enzym Faktor Xa; und schließlich
g) Abtrennen und Reinigen des solcherart erhaltenen reifen menschlichen Wachstumshormons von dem Kulturmedium.

2. Verfahren zur Herstellung einer Mutante nach Anspruch 1, worin der Aminosäurerest Arginin in Position 134 durch L-Alanin ersetzt ist.

3. Verfahren zur Herstellung einer Mutante nach Anspruch 1, worin der Aminosäurerest Arginin in Position 134 durch L-Leucin ersetzt ist.

4. Verfahren zur Herstellung einer Mutante nach Anspruch 1, worin der Aminosäurerest Arginin in Position 134 durch L-Threonin ersetzt ist.

5. Verfahren zur Herstellung einer Mutante nach Anspruch 1, worin der Aminosäurerest Arginin in Position 134 durch L-Phenylalanin ersetzt ist.

6. Verfahren zur Herstellung einer Mutante nach Anspruch 1, worin der Aminosäurerest Arginin in Position 134 durch L-Prolin ersetzt ist.

7. Verfahren zur Herstellung einer Mutante nach Anspruch 1, worin der Aminosäurerest Arginin in Position 134 durch L-Histidin ersetzt ist.

8. Verfahren zur Herstellung einer Mutante von menschlichem Wachstumshormon nach Anspruch 1, worin die Mutagenese in Stufe a) die folgenden Schritte umfaßt:
1) Insertion des hGH-Gens (Target-Sequenz) in einen Phagen vom Typ M13 oder in ein davon abgeleitetes Plasmid und Ausbildung in Einzelstrangform zur Verwendung als Matrize für die Synthese des mutanten Gens;
2) Synthese eines Oligonucleotids, das zu der zu mutagenisierenden Sequenz komplementär ist, ausgenommen einen inneren Anteil, der die Mutation bestimmt;
3) Anelierung des synthetischen Oligonucleotids an die Matrize; wobei das Oligonucleotid als Primer für die Synthese des zweiten modifizierten Stranges wirkt;
4) Rekonstitution der Doppelhelix-Zirkularstruktur mittels einer Polymerisationsstufe und in vitro-Ligation, wobei ein Strang der Parentalstrang ist, während der andere die gewünschte Mutation trägt;
5) Anwendung der Doppelhelix-Form zum Transformieren von kompetent gemachten Wirtszellen zur Ausbildung einer Population von Klonen vom Mutanten- und Wild-Typ;
6) Selektion der mutanten Klone durch Hybridisierung mit spezifischen Oligonucleotiden, Sequenzieren der erhaltenen Klone oder Restriktionsanalyse.

9. Verfahren zur Herstellung einer Mutante von menschlichem Wachstumshormon nach Anspruch 1, worin der Klonierungsvektor in Stufe b) aus der Gruppe ausgewählt ist, die aus mit Bacillus subtilis replizierbaren Expressionsplasmiden besteht.

10. Verfahren zur Herstellung einer Mutante von menschlichem Wachstumshormon nach Anspruch 1, worin der Bacillus subtilis in Stufe d) Bacillus subtilis SMS108 (pSM 386) ATCC 68657 ist.

11. Verfahren zur Herstellung einer Mutante von menschlichem Wachstumshormon nach Anspruch 1, worin der Bacillus subtilis in Stufe d) Bacillus subtilis SMS108 (pSM 387) ATCC 68658 ist.

12. Verfahren zur Herstellung einer Mutante von menschlichem Wachstumshormon nach Anspruch 1, worin der Bacillus subtilis in Stufe d) Bacillus subtilis SMS108 (pSM 388) ATCC 68661 ist.

13. Verfahren zur Herstellung einer Mutante von menschlichem Wachstumshormon nach Anspruch 1, worin der Bacillus subtilis in Stufe d) Bacillus subtilis SMS108 (pSM 389) ATCC 68662 ist.

14. Verfahren zur Herstellung einer Mutante von menschlichem Wachstumshormon nach Anspruch 1, worin der Bacillus subtilis in Stufe d) Bacillus subtilis SMS108 (pSM 390) ATCC 68660 ist.

15. Verfahren zur Herstellung einer Mutante von menschlichem Wachstumshormon nach Anspruch 1, worin der Bacillus subtilis in Stufe d) Bacillus subtilis SMS108 (pSM 391) ATCC 68659 ist.

16. Verfahren zur Herstellung einer pharmazeutischen Zusammensetzung, die zur Behandlung von Dysfunktionen der Hypophyse, Verbrennungen, Dystrophie, Pseudoarthrose und Knochenbrüchen geeignet ist, mit einem Gehalt an einer Mutante von menschlichem Wachstumshormon, erhalten gemäß Anspruch 1, als aktivem Wirkstoff.

## Revendications (Revendications pour l'(les) Etat(s) contractant(s) suivant(s): AT, BE, CH, DE, DK, FR, GB, IE, LI, LU, MC, NL, PT, SE)

1. Mutants de l'hormone de croissance humaine, stables vis-à-vis de la dégradation enzymatique et présentant une activité biologique inchangée, caractérisés en ce que le résidu d'acide aminé arginine en position 134 de la séquence d'acides aminés de l'hormone de croissance humaine naturelle est remplacé par au moins un résidu d'acide aminé choisi dans l'ensemble constitué par L-alanine, L-leucine, L-thréonine, L-phénylalanine, L-proline, L-histidine.

2. Mutant selon la revendication 1, dans lequel le résidu d'acide aminé arginine en position 134 est remplacé par un résidu de L-alanine.

3. Mutant selon la revendication 1, dans lequel le résidu d'acide aminé arginine en position 134 est remplacé par un résidu de L-leucine.

4. Mutant selon la revendication 1, dans lequel le résidu d'acide aminé arginine en position 134 est remplacé par un résidu de L-thréonine.

5. Mutant selon la revendication 1, dans lequel le résidu d'acide aminé arginine en position 134 est remplacé par un résidu de L-phénylalanine.

6. Mutant selon la revendication 1, dans lequel le résidu d'acide aminé arginine en position 134 est remplacé par un résidu de L-proline.

7. Mutant selon la revendication 1, dans lequel le résidu d'acide aminé arginine en position 134 est remplacé par un résidu de L-histidine.

8. Séquence d'ADN, comprenant le gène de structure qui code un mutant de l'hormone de croissance humaine revendiqué dans l'une des revendications 1 à 7.

9. Vecteur de clonage contenant une séquence d'ADN comportant le gène de structure qui code un mutant de l'hormone de croissance humaine revendiqué dans l'une des revendications de 1 à 7.

10. Vecteur selon la revendication 9, choisi dans l'ensemble constitué par les plasmides d'expression qui peuvent se répliquer dans Bacillus subtilis.

11. Souche de Bacillus subtilis transformée avec un plasmide selon la revendication 10.

12. Bacillus subtilis SMS 108 (pSM 386) ATCC 68657.

13. Bacillus subtilis SMS 108 (pSM 387) ATCC 68658.

14. Bacillus subtilis SMS 108 (pSM 388) ATCC 68661.

15. Bacillus subtilis SMS 108 (pSM 389) ATCC 68662.

16. Bacillus subtilis SMS 108 (pSM 390) ATCC 68660.

17. Bacillus subtilis SMS 108 (pSM 391) ATCC 68659.

18. Mutant de l'hormone de croissance humaine selon la revendication 1, obtenu par un procédé qui comporte :
a) le fait de cultiver, dans un milieu de culture approprié, une souche de Bacillus subtilis transformée par un plasmide selon la revendication 11,
b) le fait de lyser des cellules et de séparer le précurseur soluble dudit mutant, dans lequel le résidu d'acide aminé phénylalanine N-terminal est relié au nonapeptide ayant la séquence Met-Glu-Glu-Leu-Met-Ile-Glu-Gly-Arg-,
c) le fait d'hydrolyser ledit précurseur soluble avec l'enzyme Facteur Xa, et enfin,
d) le fait de séparer et de purifier le mutant de l'hormone de croissance humaine mature obtenue de cette manière.

19. Mutant de l'hormone de croissance humaine selon la revendication 18, dans lequel le Bacillus est le Bacillus subtilis SMS108 (pSM 386) ATCC 68657.

20. Mutant de l'hormone de croissance humaine selon la revendication 18, dans lequel le Bacillus est le Bacillus subtilis SMS108 (pSM 387) ATCC 68658.

21. Mutant de l'hormone de croissance humaine selon la revendication 18, dans lequel le Bacillus est le Bacillus subtilis SMS108 (pSM 388) ATCC 68661.

22. Mutant de l'hormone de croissance humaine selon la revendication 18, dans lequel le Bacillus est le Bacillus subtilis SMS108 (pSM 389) ATCC 68662.

23. Mutant de l'hormone de croissance humaine selon la revendication 18, dans lequel le Bacillus est le Bacillus subtilis SMS108 (pSM 390) ATCC 68660.

24. Mutant de l'hormone de croissance humaine selon la revendication 18, dans lequel le Bacillus est le Bacillus subtilis SMS108 (pSM 391) ATCC 68659.

25. Mutant de l'hormone de croissance humaine selon la revendication 1, destiné à être utilisé en tant que principe actif dans la préparation de compositions pharmaceutiques.

26. Composition pharmaceutique appropriée pour le traitement des dysfonctionnements de l'hypophyse, des brûlures, de la dystrophie, de la pseudarthrose et des fractures des os, contenant, comme principe actif, un mutant de l'hormone de croissance humaine selon la revendication 1.

## Revendications (Revendications pour l'(les) Etat(s) contractant(s) suivant(s): ES, GR)

1. Procédé de préparation de mutants de l'hormone de croissance humaine, stables vis-à-vis de la dégradation enzymatique et présentant une activité biologique inchangée, dans lesquels le résidu d'acide aminé arginine en position 134 de la séquence d'acides aminés de l'hormone naturelle est remplacé par au moins un résidu d'acide aminé choisi dans l'ensemble constitué par L-alanine, L-leucine, L-thréonine, L-phénylalanine, L-proline, L-histidine ; ledit procédé comprenant :
a) l'introduction d'une mutation dans le gène de structure codant l'hormone de croissance humaine, par la technique de mutagenèse dirigée ;
b) le clonage du gène qui a été soumis à la mutagenèse, obtenu dans l'étape a) dans un vecteur de clonage et d'expression pouvant se répliquer dans Bacillus subtilis.
c) la transformation d'un Bacillus subtilis avec le vecteur de recombinaison obtenu dans l'étape b) ;
d) la culture, dans un milieu de culture approprié, d'une souche de Bacillus subtilis transformée suivant l'étape c) ;
e) la lyse des cellules de Bacillus subtilis, et la séparation du surnageant contenant le précurseur soluble dudit mutant ;
f) l'hydrolyse du précurseur soluble dudit mutant avec l'enzyme Facteur Xa, et enfin,
g) la séparation, d'avec le milieu de culture, et la purification de l'hormone de croissance humaine obtenue de cette manière.

2. Procédé de préparation d'un mutant selon la revendication 1, dans lequel le résidu d'acide aminé arginine en position 134 est remplacé par un résidu de L-alanine.

3. Procédé de préparation d'un mutant selon la revendication 1, dans lequel le résidu d'acide aminé arginine en position 134 est remplacé par un résidu de L-leucine.

4. Procédé de préparation d'un mutant selon la revendication 1, dans lequel le résidu d'acide aminé arginine en position 134 est remplacé par un résidu de L-thréonine.

5. Procédé de préparation d'un mutant selon la revendication 1, dans lequel le résidu d'acide aminé arginine en position 134 est remplacé par un résidu de L-phénylalanine.

6. Procédé de préparation d'un mutant selon la revendication 1, dans lequel le résidu d'acide aminé arginine en position 134 est remplacé par un résidu de L-proline.

7. Procédé de préparation d'un mutant selon la revendication 1, dans lequel le résidu d'acide aminé arginine en position 134 est remplacé par un résidu de L-histidine.

8. Procédé de préparation d'un mutant de l'hormone de croissance humaine selon la revendication 1, dans lequel la mutagenèse de l'étape a) comprend :
1) le fait d'insérer le gène hGH (séquence cible) dans un phage de type M13 ou dans un plasmide dérivé de ce dernier, et de le préparer sous forme de simple brin, qui sera utilisé comme matrice pour la synthèse du gène mutant ;
2) la synthèse d'un oligonucléotide complémentaire de la séquence soumise à la mutagenèse, exception faite de la partie interne qui détermine la mutation ;
3) l'hybridation de l'oligonucléotide synthétique avec la matrice, l'oligonucléotide agissant en tant qu'amorce de la synthèse du second brin modifié ;
4) grâce à une étape de polymérisation et de la ligature in-vitro, la reconstitution de la structure circulaire en double hélice, dont l'un des brins représente lebrin parental tandis que l'autre porte la mutation désirée ;
5) l'utilisation de la forme en double hélice pour transformer des cellules hôtes rendues compétentes, pour obtenir une population de mutants et de clones de type sauvage ;
6) la sélection des clones de mutants par hybridation avec des oligonucléotides spécifiques, et le séquençage des clones obtenus ou leur analyse à l'aide d'enzymes de restriction.

9. Procédé de préparation d'un mutant de l'hormone de croissance humaine selon la revendication 1, dans lequel le vecteur de clonage de l'étape b) est choisi dans l'ensemble constitué par les plasmides d'expression pouvant se répliquer dans Bacillus subtilis.

10. Procédé de préparation d'un mutant de l'hormone de croissance humaine selon la revendication 1, dans lequel le Bacillus subtilis de l'étape d) est le Bacillus subtilis SMS 108 (pSM 386) ATCC 68657.

11. Procédé de préparation d'un mutant de l'hormone de croissance humaine selon la revendication 1, dans lequel le Bacillus subtilis de l'étape d) est le Bacillus subtilis SMS 108 (pSM 387) ATCC 68658.

12. Procédé de préparation d'un mutant de l'hormone de croissance humaine selon la revendication 1, dans lequel le Bacillus subtilis de l'étape d) est le Bacillus subtilis SMS 108 (pSM 388) ATCC 68661.

13. Procédé de préparation d'un mutant de l'hormone de croissance humaine selon la revendication 1, dans lequel le Bacillus subtilis de l'étape d) est le Bacillus subtilis SMS 108 (pSM 389) ATCC 68662.

14. Procédé de préparation d'un mutant de l'hormone de croissance humaine selon la revendication 1, dans lequel le Bacillus subtilis de l'étape d) est le Bacillus subtilis SMS 108 (pSM 390) ATCC 68660.

15. Procédé de préparation d'un mutant de l'hormone de croissance humaine selon la revendication 1, dans lequel le Bacillus subtilis de l'étape d) est le Bacillus subtilis SMS 108 (pSM 391) ATCC 68659.

16. Procédé de préparation d'une composition pharmaceutique appropriée pour le traitement des dysfonctionnements de l'hypophyse, des brûlures, de la dystrophie, de la pseudarthrose et des fractures des os, contenant, comme principe actif, un mutant de l'hormone de croissance humaine selon la revendication 1.
